# EUROPEAN PATENT SPECIFICATION

(11) **EP 2 566 505 B1**
(45) Date of publication and mention of the grant of the patent: **16.10.2019**
(21) Application number: 11720265.5
(22) Date of filing: 06.05.2011
(51) Int. Cl.: A61K 38/45, A61P 1/16

(54) **DELIVERY OF CHOLESTERYL ESTER TO STEROIDOGENIC TISSUES**
VERABREICHUNG VON CHOLESTERYLESTER AN STEROIDOGENE GEWEBE
ADM INISTRATION D'ESTER DE CHOLESTÉRYLE À DE TISSUS STEROIDOGÉNIQUES

(30) Priority: 06.05.2010 US 331909 P
(43) Date of publication of application: 13.03.2013
(73) Proprietor: Alphacore Pharma LLC, Ann Arbor, MI 48103 (US)
(72) Inventor: AUERBACH, Bruce. J., Ann Arbor Michigan 48105 (US); HOMAN, Reynold, Ann Arbor Michigan 48103 (US); KRAUSE, Brian, Ann Arbor Michigan 48103 (US)
(74) Representative: AstraZeneca
(86) International application number: PCT/US2011/035500
(87) International publication number: WO 2011/140429

(56) References cited:
- WO-A1-98/46767
- WO-A1-2005/051091
- WO-A1-2009/124055
- WO-A2-03/009704
- US-A1- 2003 224 041
- NG DOMINIC S ET AL: "Disruption of the murine lecithin: Cholesterol acyltransferase gene causes impairment of adrenal lipid delivery and up-regulation of scavenger receptor class B type I", JOURNAL OF BIOLOGICAL CHEMISTRY, THE AMERICAN SOCIETY OF BIOLOGICAL CHEMISTS, INC., BALTIMORE, MD, US, vol. 272, no. 25, 1 January 1997 (1997-01-01), pages 15777-15781, XP009150062, ISSN: 0021-9258

## Description

### FIELD

This disclosure relates generally to the field of medicine, and in particular, the treatment of diseases characterized by decreased stores of cholesteryl ester in steroidogenic tissues, reducing the tissue's ability to produce steroids especially during periods of demand, stress and or systemic inflammation.

### BACKGROUND

Normally, excess cholesterol is removed from tissues, such as arteries, and delivered to the liver for excretion in bile by a process known as reverse cholesterol transport (RCT). In the first step of RCT, cholesterol passes from tissue cells to high-density lipoproteins (HDL) in the circulation. In the second step, the enzyme lecithin: cholesterol acyltransferase (LCAT) enhances the cholesterol-carrying capacity of HDL by catalyzing the transesterification of a fatty acid from lecithin in HDL to cholesterol to form cholesteryl ester (CE). The cholesteryl ester product accumulates in the HDL interior until it is removed at HDL-receptors in the liver. Much of the CE entering the liver is converted to cholesterol and bile acids that are excreted in the bile. The same HDL receptor-mediated process of CE delivery occurs in steroidogenic tissues to maintain a supply of cholesterol for the production of steroids. The HDL receptor for this process is currently thought to be the scavenger receptor B1 (SR-B1).

Cholesteryl ester transfer protein (CETP) is a plasma protein responsible for the net transfer of CE from HDL to the beta-lipoproteins (VLDL and LDL). Humans deficient in CETP have greatly elevated levels of large CE-enriched HDL. Inhibitors of CETP have been shown to block the transfer of CE from HDL to LDL, thus rapidly increasing HDL-C (where C represents total cholesterol and is comprised of both cholesterol and CE) and reducing LDL-C. The resultant HDL is characteristically enriched in CE, similar to the HDL isolated from CETP deficient plasma.

The steroidogenic tissues, including adrenals, gonads (testes and ovaries) and thymus, rely on intracellular stores of cholesteryl esters as a source of cholesterol for the synthesis of steroids such as cortisol, estrogens and androgens. When needed, the ester bond is cleaved by the enzyme cholesterol esterase, liberating cholesterol for use in steroid production. In times of stress, the CE stores can be depleted quickly, and without replenishment of CE, production of steroids is greatly impaired. In vitro experiments have demonstrated that HDL isolated from CETP deficient patients can deliver more CE through SR-B1 than HDL isolated from normal subjects.

In mice, low HDL-CE results in reduced CE content in adrenals, testes and ovaries. This decreased CE content appears to impact reproduction and the ability of the adrenals to produce cortisol during periods of stress. In humans, hypoalphalipoprotenemia (low HDL-C) is a hallmark of genetic disorders such as LCAT deficiency and Tangier's disease. Hypoalphalipoprotenemia also occurs in a variety of non-genetic disorders including infection, inflammation, auto-immune diseases, arthritic diseases, sepsis, trauma, burns, liver disease (e.g. hepatitis, fibrosis, cirrhosis, bile duct hyperplasia, bile duct atresia, organ transplant, heavy metal poisoning) and kidney disease.

Although not a primary pathology in these diseases, the decreased HDL-CE reduces the delivery of cholesterol to steroidogenic tissues, thus, decreasing the body's ability to produce critical glucocorticoids, hormones and other steroids. This reduced capacity for steroidal production can exacerbate the underlying conditions. As a compounding factor, the worsening of the underlying condition can further decrease the level of HDL-CE. In some cases this cycle can lead to a life threatening condition, even death. Following trauma or severe infection patients may have reduced HDL-C levels, or may be at risk for HDL-C levels to fall. It would be useful to normalize or increase the levels of HDL-C in order to normalize or increase steroidogenesis in these patients.

Artificial forms of HDL consisting of phospholipid and apolipoprotein A-1, or of phospholipids and apolipoprotein A-1 mimetics, are being investigated for treatment of patients having cardiovascular disorders such as atherosclerosis. Such treatments are predicated upon the ability of these artificial HDLs to remove excess cholesterol from tissues such as arteries. However, because these versions of artificial HDL lack cholesterol or cholesteryl esters, they are not immediately able to deliver CE to the adrenals as is needed to normalize or increase steroidogenesis and in fact would function to remove cholesterol from tissues. In some instances, patients at risk for adrenal insufficiency have decreased levels of LCAT and therefore have a reduced capacity to make HDL-CE (the form of cholesterol used to replenish adrenals) resulting in low levels of HDL-CE. Because these artificial forms of HDL do not correct for the deficiency of LCAT or HDL-CE they do not provide an effective treatment for critical care patients at risk for adrenal insufficiency.

Thus, there is a need for treatments to increase the availability of CE to steroidogenic tissues in patients having disorders associated with decreased function of steroidogenic tissues such as the adrenals, gonads and thymus.

### SUMMARY

The compositions and uses disclosed in the present disclosure modulate the amount of steroids produced by steroidogenic tissues by normalizing or increasing HDL-CE concentrations in plasma thereby maintaining or increasing plasma levels of steroids by administering an effective dose of LCAT, or a combination of LCAT with a cholesterol delivery particle (CDP). The present disclosure describes LCAT for use in treating conditions characterized by reduced function of steroidogenic tissues. The present disclosure describes LCAT for use in treating conditions characterized by adrenal insufficiency.

### DETAILED DESCRIPTION

As used herein "adrenal insufficiency" means a condition in which the adrenal glands do not produce adequate amounts of steroid hormones. Such conditions include congenital conditions such as Addison's disease; and non-congenital adrenal insufficiency.

As used herein non-congenital adrenal insufficiency includes "acquired conditions" for example: systemic inflammatory response syndrome; infection; inflammation; sepsis; trauma; burns; liver disease, including hepatitis, hepatorenal syndrome, fibrosis, cirrhosis, bile duct hyperplasia, or bile duct atresia; kidney disease; organ transplant; or heavy metal poisoning.

Relative adrenal insufficiency also includes conditions in which steroid levels, e.g., cortisol levels, are in the normal range, yet there is an inadequate adrenal response to suppress the inflammatory response to trauma, burns, infection, or sepsis.

"Systemic inflammatory response syndrome" or "SIRS" refers to is an inflammatory state affecting the whole body and can be caused by ischemia, inflammation, trauma, infection, or a combination of several insults, for example sepsis.

The term "treating" or other forms of the word such as "treatment", or "treat" is used herein to mean that administration of a compound used in the present disclosure mitigates a disease or a disorder in a host and/or reduces, inhibits, or eliminates a particular characteristic or event associated with a disorder (e.g., reduced steroidogenesis). Thus, the term "treatment" includes, preventing a disorder from occurring in a host, particularly when the host is predisposed to acquiring the disorder; inhibiting the disorder; and/or alleviating or reversing the disorder. Insofar as the uses of the present disclosure are directed to preventing disorders, it is understood that the term "prevent" does not require that the disease state be completely thwarted. Rather, as used herein, the term preventing refers to the ability of the skilled artisan to identify a population that is susceptible to disorders, such that administration of the compounds of the present disclosure may occur prior to onset of a disease. The term does not imply that the disease state be completely avoided.

Throughout the description and claims of this specification the word "comprise" and other forms of the word, such as "comprising" and "comprises," means including, and is not intended to exclude, for example, other additives, components, integers, or steps.

As used herein, the singular forms "a", "an", and "the" include plural references unless the context clearly dictates otherwise

"Between" as used herein is inclusive, e.g., "between 1 mg and 5000 mg" includes 1 mg and 5000 mg.

"From" as used herein is inclusive, e.g., "from 1 mg to 5000 mg" includes 1 mg and 5000mg.

"SC" means subcutaneous injection.

"IV" means intravenous injection or infusion.

"IM" means intramuscular injection.

"FC" is an abbreviation for free cholesterol and as used herein means non-esterified cholesterol.

"CE" is an abbreviation for cholesteryl ester

"CDP" and "cholesteryl ester delivery particle" are used interchangeably and as used herein, means a molecule that is able to provide CE to steroidogenic tissue, including: a complex of apolipoprotein AI, phospholipid and cholesteryl ester; a complex of apolipoprotein AI, phospholipid, cholesterol, and cholesteryl ester; a complex of one or more amphipathic peptide of 18- 40 amino acids in length, phospholipid and cholesteryl ester; a complex of one or more amphipathic peptides of 18- 40 amino acids in length, phospholipid, cholesterol and cholesteryl ester; or native HDL. Phospholipids suitable for use in producing CDP include phosphatidylcholine, sphingomyelin, phosphatidylethanolamine, phosphatidylserine, phosphatidyinositol, phosphatidylglycerol, cardiolipin and mixtures thereof. The term CDP encompasses rHDL, mHDL, and native HDL.

"rHDL" and "reconstituted HDL" are used interchangeably and as used herein, means a complex of apolipoprotein AI, phospholipid and cholesteryl ester; or a complex of apolipoprotein AI, phospholipid, cholesterol, and cholesteryl ester. Phospholipids suitable for use in producing rHDL include phosphatidylcholine, sphingomyelin, phosphatidylethanolamine, phosphatidylserine, phosphatidyinositol, phosphatidylglycerol, cardiolipin and mixtures thereof.

"mHDL" and "mimetic HDL", are used interchangeably and as used herein means a complex of one or more amphipathic peptide of 18- 40 amino acids in length, phospholipid and cholesteryl ester; or a complex of one or more amphipathic peptides of 18-40 amino acids in length, phospholipid, cholesterol and cholesteryl ester. Phospholipids suitable for use in producing mHDL include phosphatidylcholine, sphingomyelin, phosphatidylethanolamine, phosphatidylserine, phosphatidyinositol, phosphatidylglycerol, cardiolipin and mixtures thereof. Amphipathic peptides suitable for use in producing CDP include D4F (Song et. al, (2009) Int J Biol Sci 5:637-646), (A.V. Bocharov et al.(2004) J. Biol. Chem. 279: 36072-36082), 5A and analogs (W. D'Souza et al (2010) Circ. Res. 107:217-227), A-IConA (G.M. Anantharamaiah (2007) J. Lipid Res. 48:1915-1923), trimeric apoAI variants (Graversen, et al, 2008); Ac-hE18A-NH2 (Datta, et al, 2000; 2001), and peptides disclosed in D Busseuil et al. (2008) Br J Pharmacal. 154(4): 765-773.

"Native HDL" means HDL isolated from plasma. These particles contain phospholipid, proteins, cholesterol and cholesteryl ester.

As used herein, a "normal level of HDL-CE" means a plasma concentration of HDL-CE that is present in an average healthy untreated subject not currently on any medication which might alter HDL-CE levels.

As used herein "a normal level of a steroid" means a plasma concentration of a particular steroid that is present in an average healthy untreated subject not currently on any medication which might alter the level of that particular steroid.

"LCAT" is an abbreviation for lecithin-cholesterol acyltransferase.

"LCAT" or "LCAT polypeptide" when used herein encompass native sequence LCAT, LCAT variants, modified LCAT, LCAT derivatives and chimeric LCAT. A "native sequence LCAT" comprises a polypeptide having the same amino acid sequence as a LCAT derived from nature. Thus, a native sequence LCAT specifically encompasses naturally occurring truncated forms of LCAT, and naturally-occurring allelic variants of LCAT, naturally-occurring variant forms (e.g., alternately spliced forms). The preferred native sequence LCAT is a mature native sequence LCAT.

"Modified LCAT" means a polypeptide which has LCAT activity, wherein one or more amino acids in the native LCAT polypeptide is substituted with another amino acid, or one or more amino acids is added, or one or more amino acids is deleted from, to a portion of the native polypeptide, including the N-terminal or C-terminal amino acid. Examples of substitutions include conservative amino acid substitutions, or substitutions with non-naturally occurring amino acids. Non-limiting exemplary conservative amino acid substitutions are provided in Table 1. For example the modified LCAT may be a modified LCAT protein as described in United States Patent Publication No. 2009/0081182.

Derivatives of LCAT include native or modified LCAT polypeptides which have been altered to improve the activity, solubility, absorption, and/or biological half life. One of skill in the art would be familiar with methods for derivatizing polypeptides to improve their pharmacologic properties.

**TABLE 1**

| Original Residue | Exemplary Conservative Substitutions |
|---|---|
| A | G, S |
| R | K |
| N | Q, H |
| D | E |
| C | S |
| Q | N |
| E | D |
| G | A, P |
| H | N, Q |
| I | L, V |
| L | I, V |
| K | R, Q, E |
| M | L, Y, I |
| F | M, L, Y |
| S | T |
| T | S |
| W | Y |
| Tyr | W, F |
| Val | I, L |

"ApoA-1", "ApoA-I polypeptide" and "Apolipoprotein A-1" are used interchangeably herein.

"ApoA-1" as used herein encompasses native sequence apoA-1, modified apoA-1, apoA-1 derivatives, and chimeric apoA-1.

A "native sequence apoA-1" comprises a polypeptide having the same amino acid sequence as an apoA-1 polypeptide derived from nature. Thus, a native sequence apoA-1 specifically encompasses naturally occurring truncated forms of apoA-1, and naturally-occurring allelic variants of apoA-1, naturally-occurring variant forms (e.g., alternately spliced forms), and pre-pro or pro- apoA-1. The preferred native sequence apoA-1 is a mature native sequence apoA-1.

"Modified apoA-1" means a polypeptide having an amino acid sequence which differs from a native apoA-1 polypeptide sequence in that one or more amino acids is substituted with a different amino acid, or one or more amino acids is added, or one or more amino acids is deleted and wherein the polypeptide retains the ability to form a CDP. Examples of such substitutions include conservative substitutions, or substitutions with non-naturally occurring amino acids.

Derivatives of apoA-1 include native or modified Apo A-I polypeptides which have been altered to improve the solubility, absorption, biological half life and wherein the derivative retains the ability to form a CDP. Derivatives of polypeptides are well known in the art e.g., pegylation. One of skill in the art would be familiar with methods for derivatizing polypeptides to improve their pharmacologic properties.

HDL-C refers to HDL which comprises cholesterol with or without cholesteryl ester. (where C represents total cholesterol and is comprised of both cholesterol and cholesteryl ester)
HDL-CE refers to the cholesteryl ester component of HDL.

The term "effective amount", as used herein, means an amount of LCAT or a combination of LCAT with a CDP, effective at dosages and for periods of time necessary to achieve the desired or therapeutic result. An effective amount may vary according to factors known in the art, such as the disease state, age, sex, and weight of the subject being treated. Although particular dosage regimens may be described in examples herein, a person skilled in the art would appreciate that the dosage regimen may be altered to provide optimum therapeutic response. For example, several divided doses may be administered daily or the dose may be proportionally reduced as indicated by the exigencies of the therapeutic situation. In addition, the compositions of this disclosure can be administered as frequently as necessary to achieve the optimum therapeutic response.

Lipids suitable for use in producing the compositions of the present disclosure include natural and synthesized (synthetic) lipids and phospholipids, phosphatidylcholine, sphingomyelin, phosphatidylethanolamine, phosphatidylserine, phosphatidyinositol, phosphatidylglycerol, cardiolipin; phosphatidylcholine in which a fatty acid is esterified to the SN-1 position of the glycerol backbone of phosphatidylcholine in which the fatty acids include myristic, palmitic, palmitoleic, oleic and stearic acids; phosphatidylcholine in which a fatty acid is esterified to the SN-2 position of the glycerol backbone of phosphatidylcholine, in which the fatty acids include myristic, palmitic, palmitoleic, oleic and stearic acids; phosphatidylcholine in which the SN-1 or SN-2 positions of the glycerol backbone is linked by ether bonds to fatty alcohols including tetradecanol, hexadecanol and octadecanol; included also are phospholipids as described wherein the moiety esterified to the phosphate group in place of choline is ethanolamine, serine, glycerol or inositol.

Subjects having conditions associated with adrenal insufficiency often have decreased levels of HDL-CE. Such conditions, include systemic inflammatory response syndrome, infection, inflammation, auto-immune diseases, arthritic diseases, sepsis, trauma, burns, liver disease (e.g. hepatitis, fibrosis, cirrhosis, bile duct hyperplasia, bile duct atresia, organ transplant, heavy metal poisoning), and kidney disease.

Although not a primary pathology in these diseases, the decreased HDL-CE reduces the delivery of cholesterol to steroidogenic tissues, thus, decreasing the body's ability to produce critical glucocorticoids, hormones and other steroids. The adrenal gland does not store cortisol, it relies on cholesterol derived from the cholesteryl ester that is supplied by HDL-CE for synthesis of cortisol. This reduced capacity for steroidal production can exacerbate of the underlying conditions leading to serious complications. The present disclosure provides LCAT for use in prophylactically elevating HDL-CE levels in patients who may have normal to elevated HDL-C when presenting following trauma, severe infection or prior to major surgery, in order to prevent the significant drop in HDL-C that routinely occurs in these patients. In addition, the present disclosure provides LCAT for use in treating patients currently have reduced HDL-C levels, following trauma or severe infection in order to prevent a decreased level of HDL-C levels.

LCAT is responsible for the production of HDL-CE through the esterification of the free cholesterol component of HDL-C. Thus, increasing LCAT levels or LCAT activity increases the amount of HDL-CE available for delivery to steroidogenic tissues. For example, to the adrenal glands thereby allowing for replenishment of the adrenal cholesterol stores which are used in the production of corticosteroids. The LCAT level and/or LCAT activity can be increased by any means available.

Accordingly, the present disclosure provides LCAT for use in modulating lipid content of steroidogenic tissues in a patient suffering from a condition wherein the function of steroidogenic tissues is reduced, wherein said condition is an acute condition as defined below. In one embodiment the lipid content is modulated by administering an effective dose of LCAT. In another embodiment the lipid content is modulated by administering in combination, two or more agents where one agent is LCAT, and the other agent or agents are independently selected from native HDL, rHDL, and mHDL. The two or more agents can be administered in any order, for example, simultaneously or sequentially.

An embodiment of the present disclosure is LCAT for use in increasing steroidogenesis of a steroid in a patient suffering from a condition wherein the function of steroidogenic tissues is reduced, wherein said condition is an acute condition as defined below whereby the steroid level is increased. In one embodiment the steroid is a corticosteroid or sex hormone. In another embodiment the steroid is cortisol. In a further embodiment the steroid is aldosterone. In yet another embodiment the steroid is testosterone. In still another embodiment the steroid is an estrogen. A further embodiment of the present disclosure is LCAT for use in increasing steroidogenesis in a patient in need thereof whereby the steroid level is increased to about a normal level. Another embodiment of the present disclosure is LCAT for use in increasing steroidogenesis in a patient in need thereof, whereby the steroid level is increased to above a normal level.

One embodiment of the present disclosure is LCAT for use in increasing cortisol in a patient suffering from a condition wherein the function of steroidogenic tissues is reduced, wherein said condition is an acute condition as defined below.

Another embodiment according to the present disclosure is LCAT for use in treating a condition characterized by reduced function of steroidogenic tissues, wherein the condition is an acute condition selected from those listed below. In one embodiment the acute condition is systemic inflammatory response syndrome. In another embodiment the acute condition is infection. In another embodiment the condition is inflammation. In yet another embodiment the acute condition is sepsis. In still another embodiment the acute condition is trauma. In another embodiment the acute condition is burn. In yet another embodiment the acute condition is liver disease. In still another embodiment the acute condition is kidney disease. In another embodiment the acute condition is organ transplant. In yet another embodiment the acute condition is heavy metal poisoning.

Liver disease includes hepatitis, hepatorenal syndrome, fibrosis, cirrhosis, bile duct hyperplasia, or bile duct atresia. Thus, one embodiment of the present disclosure is LCAT for use in treating a liver disease wherein the liver disease is hepatitis, hepatorenal syndrome, fibrosis, cirrhosis, bile duct hyperplasia, or bile duct atresia. In a particular embodiment the liver disease is hepatitis. In another embodiment the liver disease is hepatorenal syndrome. In yet another embodiment the liver disease is fibrosis. In still another embodiment the liver disease is cirrhosis. In another embodiment the liver disease is bile duct hyperplasia. In another embodiment the liver disease is bile duct atresia.

In certain conditions, it may be desirable to immediately increase the amount of HDL-CE to a normal or above normal level and then to maintain a normal or increased level of HDL-CE. Such conditions are generally acute conditions such as sepsis, major surgical trauma, or severe burns. One of skill in the art would understand which conditions would require an immediate increase in the amount of HDL-CE. Thus, an embodiment of the present disclosure comprises administrating, to a subject in need thereof, an effective amount of a CDP as an initial dose and simultaneously or subsequently administering to the subject
an amount of LCAT sufficient to maintain a desired level of HDL-CE. In preferred embodiments the desired plasma level of HDL-C is greater than 35 mg/dl.

Another embodiment is LCAT for use in treating a condition which is characterized by adrenal insufficiency or can lead to adrenal insufficiency, wherein an effective amount of a CDP is administered to a patient in need thereof as an initial dose and then an amount of LCAT sufficient to maintain a desired level of HDL-CE is administered. A particular embodiment is LCAT for use in treating sepsis wherein an effective amount of a CDP is administered to a patient in need thereof as an initial dose and then an amount of LCAT sufficient to maintain a desired level of HDL-CE is administered. In preferred embodiments the desired plasma level of HDL-C is greater than 35 mg/dl.

Patients who develop systemic inflammatory response syndrome (SIRS) frequently have reduced levels of HDL-CE. Those patients with reduced levels of HDL-CE have a worse prognosis than those with normal levels of HDL-CE. Thus, an embodiment of the disclosure is LCAT for use in treating systemic inflammatory response syndrome wherein an effective amount of LCAT is administered to a patient with systemic inflammatory response syndrome. In a particular embodiment the effective amount of LCAT is an amount sufficient to maintain the normal level of HDL-CE. In another embodiment the effective amount of LCAT is an amount sufficient to increase the level of HDL-CE above the normal level. Conditions related to SIRS include sepsis, infection, trauma (including surgery), burns and pancreatitis. Thus, an embodiment of the present disclosure is LCAT for use in treating a patient with sepsis, infection, trauma, surgical trauma, burn, or pancreatitis wherein an effective amount of LCAT is administered.

A person of ordinary skill in the art, such as a physician, would understand that, depending on the condition and severity of the condition, that different combinations of LCAT and CDP could be administered. The person of ordinary skill in the art would be able to determine which combination would be appropriate. For example a physician may decide to administer LCAT with a CDP depending on the severity of crisis.

LCAT according to the present disclosure, can be used in combination therapy with other drugs. Such therapies include simultaneous or sequential administration of the drugs involved. For example, LCAT formulations can be administered with drugs that are commonly used as a standard of care for a particular condition.

### Formulations

LCAT administered in the uses of the present disclosure can be formulated as pharmaceutical compositions and administered to a mammalian subject, such as a human patient in a variety of forms adapted to the chosen route of administration, i.e., orally, parenterally, by intravenous, intramuscular or subcutaneous routes.

Pharmaceutical compositions suitable for the delivery of compounds of the present disclosure and methods for their preparation will be readily apparent to those skilled in the art. Such compositions and methods for their preparation may be found, for example, in Remington's Pharmaceutical Sciences, 19th Edition (Mack Publishing Company, 1995).

Suitable dosage forms for oral administration include, for example, solid, semi-solid and liquid systems such as in hard or soft shell gelatin capsules, tablets, liquids, powders, lozenges (including liquid-filled), chews, gels, films, ovules, sprays, elixirs, suspensions, syrups, buccal/mucoadhesive patches and the like.

Oral dosage forms may, for example, contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active compound may be incorporated into sustained-release preparations and devices. The active compound may also be administered intravenously or intraperitoneally by infusion or injection. Solutions of the active compound or its salts can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations contain a preservative to prevent the growth of microorganisms.

The pharmaceutical dosage forms suitable for injection or infusion can include sterile aqueous solutions or dispersions or sterile powders comprising the active ingredient which are adapted for the extemporaneous preparation of sterile injectable or infusible solutions or dispersions, optionally encapsulated in liposomes. In all cases, the ultimate dosage form must be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate and gelatin.

### Dosage

In an embodiment of the present disclosure LCAT is administered by subcutaneous injection. In another embodiment LCAT is administered by intramuscular injection. In another embodiment LCAT is administered by intravenous injection or infusion. In some embodiments the LCAT is self-administered by the patient either by subcutaneous or intramuscular injection. Self-administration of LCAT is a preferred embodiment for chronic treatment, including auto-immune diseases, arthritic diseases, and chronic liver disease.

An effective daily dose of LCAT is from 1 mg to 5000 mg, or 1 mg to 2000 mg, or 10 mg to 5000 mg, or 10 mg to 1000 mg, 10 mg to 500 mg or 5 to 100 mg.

In an embodiment of the present disclosure a CDP is administered by subcutaneous injection. In another embodiment a CDP is administered by intramuscular injection. In another embodiment a CDP is administered by intravenous injection or infusion. An effective amount of a CDP is between 100 mg and 5000 mg, or between 200 mg and 20,000 mg, or between 250 mg and 10,000 mg or between 250 mg and 2000 mg, or between 500 mg and 5000 mg, between 250 mg and 1000 mg, or between 500 mg and 5000 mg or between 500 mg and 10,000 mg. The specific dosage used can vary. For example, the dosage can depend on a number of factors including the dosing frequency, the specific activity of the recombinant LCAT enzyme, the body weight of the patient, special requirements of the patient, special conditions of the patient (e.g., abnormal kidney or liver function), the condition being treated, etc. The dosing frequency and amount may, at the physician's discretion, fall outside of the typical range given herein. These dosages are based on an average human subject having a weight of 70kg. Determination of optimum dosages for a particular patient is well-known to those skilled in the art. The physician will readily be able to determine doses for subjects whose weight falls outside this range, such as infants and the elderly.

Depending on the disorder and the patient being treated, one skilled in the art (i.e. a physician) could determine the appropriate dose of the agent or agents depending on the condition of the patient.

The efficacy of a particular dose may be assessed by reference noting the change in HDL-C or improvement in certain physiologic parameters. Suitable physiologic parameters include improved cortisol response to ACTH, improved cortisol levels, change in circulating levels of steroids, e.g., corticosteroids, glucocorticoids, cortisol, testosterone, estrogen, decreased inflammatory cytokines, and improved liver function.

For example the therapeutically effective dose of one or a combination of agents may be the amount necessary to increase levels of HDL-C and/or maintain levels of HDL-C at or above 30 mg/dl, or at or above 35 mg/dl, or at or above 40 mg/dl, or at or above 45 mg/dl, or at or above 50 mg/dl. Preferably the level of HDL-C is at or above 35 mg/dl. Or for example the therapeutically effective amount of one or a combination of agents may be the amount required to raise or maintain cortisol levels at or above 15µg/dl, or at or above 20 µg/dl, or at or above 25 µg/dl or at or above 30 µg/dl or at or above 35 µg/dl. Preferably the cortisol level is at or above 25 µg/dl.

Measurement of biomarker levels and parameters described above may be measured using methods that are well known in the art. For example, change in HDL-CE level is easily detected with standard clinical laboratory procedures. Likewise, measuring the adrenal response to ACTH is commonly used in patients with suspected adrenal insufficiency. One of ordinary skill in the art would understand the significance of the results and may choose to adjust the dose based on assessments such as those described above.

Embodiments of compounds, compositions and uses are illustrated in the following examples.

### Assays

Efficacy of an agent of the present disclosure can be determined by any suitable method. For example, one method for determining the activity of an agent or composition according to the present disclosure is by using an ACTH stimulation assay which measures the adrenal response to adrenocorticotropic hormone (ACTH) by measuring cortisol production under various experimental conditions. For example, intramuscular injection of rabbits with croton oil causes an acute inflammatory response and a reduction of plasma HDL by greater than 50% within 3-days after injection. The observed inflammatory response and reduction of HDL are similar to the conditions observed in critical care patients at risk for developing adrenal insufficiency. Using this rabbit model, an efficacy of an agent of the present disclosure can be evaluated by measuring the recovery of cortisol response in stressed rabbits with low HDL in response to administration of the agent. The following experimental procedure is provided as a specific example.

Three days prior to treatment, twenty-four (24) rabbits are injected with 0.1 ml ACTH (1mg/ml), and cortisol measured at 0.5, 1, 2, and 3 hours post-stimulus. The animals are then are allocated, based on cortisol response to the ACTH challenge, into groups as follow: negative control group (saline), positive control group (croton oil only), croton oil plus 10 mg/kg LCAT, croton oil plus 30 mg/kg LCAT, croton oil plus 50 mg/kg LCAT, croton oil plus 10 mg/kg rHDL, croton oil plus 30 mg/kg rHDL, croton oil plus 50 mg/kg rHDL. At time = 0 each group is administered IM injections of croton oil or saline (negative control group). At 72 and 96 hours, animals are treated with saline or LCAT or rHDL. At 98 hours (2 hours post last treatment), all animals are injected with 0.1 ml ACTH (1mg/ml), and cortisol is measured at 0.5, 1, 2, and 3 hours post ACTH challenge. The animals in the positive control group (croton oil only) are expected to show a markedly reduced production of cortisol in comparison with the negative control group. The efficacy of the agent at each dose is determined by comparison with the cortisol levels of the control groups.

Similarly, the effect of agents according to the present disclosure may be assessed for efficacy in treating low testosterone production or low ovarian hormone production using assays known in the art.

### EXAMPLES

### Example 1.

A patient is admitted into the hospital for major surgery. The patients HDL-C level is 25 mg/dl. Based on epidemiological studies, this patient is at a significantly increased risk for infection and in-hospital death following surgery. The patient is administered a dose of LCAT of 40 mg 24 hours prior to surgery. The patient's HDL-C is measured prior to surgery, and is now above 50 mg/dl, thus greatly reducing risk of infection and/or death following the procedure.

### Example 2.

A burn victim is stabilized in the hospital with an HDL-C level of 12 mg/dl. Based on epidemiological studies, this patient's risk of going into shock and dying is greatly increased over the next few days due to the reduced ability of his adrenals to manufacture corticosteroids. The patient is infused with rHDL at a dose of 25 mg/kg over a period of 5 hours, raising the patient's HDL-C by 50 mg/dl. In order to maintain this level of HDL, the patient is administered a 60 mg dose of LCAT. Cortisol levels are monitored periodically to determine if the adrenal function is normalized. If adrenal function is still reduced, an additional dose of rHDL or LCAT may be administered.

### Example 3.

A victim of a car accident with abdominal injuries is brought to the hospital. Her HDL is 55 mg/dl. As HDL routinely falls after trauma, the physician prescribes 150 mg/day anacetrapib and an injection of LCAT (40 mg) every 5 days until the patient is fully stabilized. This treatment maintained the patient's HDL at >75 mg/dl throughout her recovery. Adrenal function was maintained.

### Example 4.

A patient is beginning to enter septic shock. Cortisol levels are <25µg/dl. The patient is infused with native HDL at a dose of 25 mg/kg over a period of 5 hours. The patient's HDL-C level is increased by 50 mg/dl. In order to maintain this level of HDL-C, the patient is administered a 60 mg bolus of LCAT. To aid in maintaining the HDL-CE, the patient is also given torcetrapib (120 mg b.i.d.). The patient is maintained on daily torcetrapib and a weekly LCAT injection during recovery. Cortisol levels are monitored to determine if the adrenal function is normalized. Dosages of torcetrapib and LCAT are adjusted as required.

### Example 5.

### Preparation of rHDL.

An aqueous suspension of 1 mole part phosphatidylcholine, 0.2 mole part cholesterol and 0.1 mole part cholesteryl ester is sonicated in an aqueous solution containing apolipoprotein A-I as described in Song et al, Int J Biol Sci 5:637-646. The lipids are co-dissolved in a solvent or solvent mixture. Solvents are removed by evaporation and vacuum. An aqueous solution of apolipoprotein A-I is added to the dried lipids (at 1 mole part apolipoprotein A-I to every 100 mole parts phospholipid.) The protein and lipids are dispersed by ultrasonication yielding rHDL.

### Example 6.

### Alternate process for the preparation of rHDL.

A lyophilized cake of phosphatidylcholine, cholesterol and cholesteryl ester is rehydrated with an aqueous solution containing apolipoprotein A-I. The cake consists of phospholipid, cholesterol and cholesteryl ester in a mole ratio of 1 mole part phosphatidylcholine, 0 to 0.5 mole parts cholesterol and 0 to 0.5 mole parts cholesteryl ester, as above. The lyophilized cake is formed by dissolving all lipids in a solvent that will form a solid at a temperature suitable for lyophilization (e.g. dioxane). The lyophilized cake is rehydrated in an aqueous solution of apolipoprotein A-I containing 1 mole part apolipoprotein A-I for every 25 to 250 mole parts phospholipid. Homogenization techniques such as heating, ultrasonication or passage through micropore filters may be necessary to obtain a stable and uniform size of rHDL.

### Example 7.

### Preparation of mHDL.

A lyophilized cake of 1 mole part phosphatidylcholine, 0 to 0.5 mole parts cholesterol, 0 to 0.5 mole parts cholesteryl ester and 0.1 to 1 mole part peptide are dissolved in dioxane alone or with an additional solvent such as methanol or acetic acid to aid in peptide dissolution. The solution is lyophilized. The resultant dried cake is reconstituted in an aqueous salt solution. The reconstitution process may require homogenization techniques such as heating, ultrasonication or passage through micropore filters to obtain a stable and uniform size of mHDL.

## Claims

1. Lecithin: cholesterol acyltransferase (LCAT) for use in the treatment of a patient having a condition wherein the function of steroidogenic tissues is reduced, wherein the condition is an acute condition selected from the group consisting of infection, sepsis, trauma, burns, liver disease, organ transplant, or heavy metal poisoning.

2. LCAT for use in increasing steroidogenesis of a steroid in a patient suffering from a condition wherein the function of steroidogenic tissues is reduced, wherein said condition is an acute condition selected from the group consisting of infection, sepsis, trauma, burns, liver disease, organ transplant, or heavy metal poisoning.

3. LCAT for use according to claim 2, wherein the steroid is a corticosteroid or sex hormone.

4. LCAT for use according to claim 3, wherein the steroid is cortisol, aldosterone, testosterone or an estrogen.

5. LCAT for use according to any one of claims 1-4, in combination with a cholesterol delivery particle (CDP).

6. LCAT for use according to claim 5, wherein the CDP is native HDL, reconstituted HDL or mimetic HDL, wherein
native HDL means HDL isolated from plasma;
reconstituted HDL means a complex of apolipoprotein Al, a phospholipid and a cholesteryl ester; or a complex of apolipoprotein Al, a phospholipid, cholesterol and a cholesteryl ester; and
mimetic HDL means a complex of one or more amphipathic peptides of 18-40 amino acids in length, a phospholipid and a cholesteryl ester; or a complex of one or more amphipathic peptides of 18-40 amino acids in length, a phospholipid, cholesterol and a cholesteryl ester.

7. LCAT for use according to claim 5 or claim 6, wherein the CDP is administered in a bolus dose prior to administration of the LCAT.

8. LCAT for use according to any one of claims 2-4, wherein the steroid level is increased to a normal level.

9. LCAT for use according to any one of claims 2-4, wherein the steroid level is increased to above a normal level.

10. LCAT for use according to any one of claims 1-6, wherein the condition is a liver disease selected from the group consisting of hepatitis, hepatorenal syndrome, fibrosis, cirrhosis, bile duct hyperplasia, or bile duct atresia.

## Patentansprüche

1. Lecithin-Cholesterin-Acetyltransferase (LCAT) zur Verwendung bei der Behandlung eines Patienten mit einem Leiden, bei dem die Funktion steroidogener Gewebe vermindert ist, wobei es sich bei dem Leiden um ein akutes Leiden ausgewählt aus der aus Infektion, Sepsis, Trauma, Verbrennungen, Leberkrankheit, Organtransplantation oder Schwermetallvergiftung bestehenden Gruppe handelt.

2. LCAT zur Verwendung beim Erhöhen der Steroidogenese eines Steroids in einem an einem Leiden leidenden Patienten, bei dem die Funktion steroidogener Gewebe vermindert ist, wobei es sich bei dem Leiden um ein akutes Leiden ausgewählt aus der aus Infektion, Sepsis, Trauma, Verbrennungen, Leberkrankheit, Organtransplantation oder Schwermetallvergiftung bestehenden Gruppe handelt.

3. LCAT zur Verwendung nach Anspruch 2, wobei es sich bei dem Steroid um ein Corticosteroid oder ein Sexualhormon handelt.

4. LCAT zur Verwendung nach Anspruch 3, wobei es sich bei dem Steroid um Cortisol, Aldosteron, Testosteron oder ein Estrogen handelt.

5. LCAT zur Verwendung nach einem der Ansprüche 1-4 in Kombination mit einem Cholesterinverabreichungspartikel (Cholesterol Delivery Particle, CDP).

6. LCAT zur Verwendung nach Anspruch 5, wobei es sich bei dem CDP um natives HDL, rekonstituiertes HDL oder mimetisches HDL handelt, wobei
natives HDL aus Plasma isoliertes HDL bedeutet,
rekonstituiertes HDL einen Komplex aus Apolipoprotein A1, einem Phospholipid und einem Cholesterylester oder einen Komplex aus Apolipoprotein A1, einem Phospholipid, Cholesterin und einem Cholesterylester bedeutet und
mimetisches HDL einen Komplex von einem oder mehreren amphipathischen Peptiden mit einer Länge von 18-40 Aminosäuren, einem Phospholipid und einem Cholesterylester oder einen Komplex von einem oder mehreren amphipathischen Peptiden mit einer Länge von 18-40 Aminosäuren, einem Phospholipid, Cholesterin und einem Cholesterylester bedeutet.

7. LCAT zur Verwendung nach Anspruch 5 oder Anspruch 6, wobei das CDP als Bolusdosis vor der Verabreichung des LCAT verabreicht wird.

8. LCAT zur Verwendung nach einem der Ansprüche 2-4, wobei der Steroidspiegel auf einen normalen Spiegel erhöht wird.

9. LCAT zur Verwendung nach einem der Ansprüche 2-4, wobei der Steroidspiegel auf über einen normalen Spiegel erhöht wird.

10. LCAT zur Verwendung nach einem der Ansprüche 1-6, wobei es sich bei dem Leiden um eine aus der aus Hepatitis, hepatorenalem Syndrom, Fibrose, Zirrhose, Gallengangshyperplasie und Gallengangsatresie bestehenden Gruppe ausgewählte Leberkrankheit handelt.

## Revendications

1. Lécithine-cholestérol acyltransférase (LCAT) pour une utilisation dans le traitement d'un patient possédant une affection où la fonction des tissus stéroïdogènes est réduite, l'affection étant une affection aiguë choisie dans le groupe constitué par une infection, une septicémie, un traumatisme, des brûlures, une maladie du foie, une transplantation d'organe ou un empoisonnement par des métaux lourds.

2. LCAT pour une utilisation dans l'augmentation de la stéroïdogenèse d'un stéroïde chez un patient souffrant d'une affection, la fonction des tissus stéroïdogènes étant réduite, ladite affection étant une affection aiguë choisie dans le groupe constitué par une infection, une septicémie, un traumatisme, des brûlures, une maladie du foie, une transplantation d'organe ou un empoisonnement par des métaux lourds.

3. LCAT pour une utilisation selon la revendication 2, le stéroïde étant un corticostéroïde ou une hormone sexuelle.

4. LCAT pour une utilisation selon la revendication 3, le stéroïde étant le cortisol, l'aldostérone, la testostérone ou un oestrogène.

5. LCAT pour une utilisation selon l'une quelconque des revendications 1-4, en combinaison avec une particule de libération de cholestérol (CDP).

6. LCAT pour une utilisation selon la revendication 5, la CDP étant une HDL (high density lipoprotein - lipoprotéine à haute densité) native, une HDL reconstituée ou une HDL mimétique,
une HDL native signifiant une HDL isolée à partir de plasma ;
une HDL reconstituée signifiant un complexe d'une apolipoprotéine AI, d'un phospholipide et d'un ester de cholestéryle ; ou un complexe d'une apolipoprotéine AI, d'un phospholipide, de cholestérol et d'un ester de cholestéryle ; et
une HDL mimétique signifiant un complexe d'un ou plusieurs peptide(s) amphipathique(s) d'une longueur de 18-40 acides aminés, d'un phospholipide et d'un ester de cholestéryle ; ou un complexe d'un ou plusieurs peptide(s) amphipathique(s) d'une longueur de 18-40 acides aminés, d'un phospholipide, de cholestérol et d'un ester de cholestéryle.

7. LCAT pour une utilisation selon la revendication 5 ou la revendication 6, la CDP étant administrée en une dose de bolus avant l'administration de la LCAT.

8. LCAT pour une utilisation selon l'une quelconque des revendications 2-4, le niveau de stéroïde étant augmenté jusqu'à un niveau normal.

9. LCAT pour une utilisation selon l'une quelconque des revendications 2-4, le niveau de stéroïde étant augmenté jusqu'à un niveau supérieur au niveau normal.

10. LCAT pour une utilisation selon l'une quelconque des revendications 1-6, l'affection étant une maladie du foie choisie dans le groupe constitué par l'hépatite, le syndrome hépatorénal, la fibrose, la cirrhose, l'hyperplasie des canaux biliaires ou l'atrésie des canaux biliaires.
